## Europäisches Patentamt
## European Patent Office
## Office européen des brevets

(11) Publication number: **0 260 030 B1**

# EUROPEAN PATENT SPECIFICATION

(45) Date of publication of patent specification: **04.11.92**  (51) Int. Cl.⁵: **A61K 7/32**

(21) Application number: **87307588.1**

(22) Date of filing: **27.08.87**

The file contains technical information submitted after the application was filed and not included in this specification

(54) **Transparent deodorant stick.**

(30) Priority: **29.08.86 GB 8620895**

(43) Date of publication of application:
**16.03.88 Bulletin 88/11**

(45) Publication of the grant of the patent:
**04.11.92 Bulletin 92/45**

(84) Designated Contracting States:
**AT BE CH DE ES FR GB GR IT LI NL SE**

(56) References cited:
**GB-A- 2 062 466**
**US-A- 4 518 582**

**CHEMICAL ABSTRACTS, vol. 82, no. 12, 24 March 1975, Columbus, OH (US); p. 262, no. 77008j**

**CHEMICAL ABSTRACTS, vol. 99, no. 12, 19 September 1983, Columbus, OH (US); p. 333, no. 93511z**

(73) Proprietor: **UNILEVER PLC**
**Unilever House Blackfriars P.O. Box 68**
**London EC4P 4BO(GB)**
(84) Designated Contracting States:
**GB**

(73) Proprietor: **UNILEVER N.V.**
**Burgemeester s'Jacobplein 1 P.O. Box 760**
**NL-3000 DK Rotterdam(NL)**
(84) Designated Contracting States:
**BE CH DE ES FR GR IT LI NL SE AT**

(72) Inventor: **Pettigrew, Robert**
**18 Brancote Gardens**
**Bromborough Wirral L62 6AH(GB)**

(74) Representative: **Elliott, Peter William et al**
**Unilever plc, Patent Division Colworth House Sharnbrook**
**Bedford MK44 1LO(GB)**

**Description**

FIELD OF INVENTION

The invention relates to transparent deodorant sticks for topical application to human skin.

BACKGROUND AND PRIOR ART

In the manufacture of deodorant cosmetic sticks which are required to be totally transparent, problems can arise when it is necessary to store these sticks prior to sale or use. In particular, shrinkage due to loss of volatile ingredients can occur with the consequence that the sticks can become misshapen and brittle as they dry out. They can also become opaque. In consequence of these problems, no transparent sticks are generally commercially available.

It has been proposed in Japanese Patent Kokai No.49/85250 of Tombo Pencil Co., to provide solid perfumery products in the form of a stick containing a sorbitol/benzaldehyde condensation product, ethanol water and propylene glycol, in addition to a substantial amount of perfume. There is also described in this reference a formulation in which the propylene glycol is replaced with glycerol together with hydroxypropylcellulose which is incorporated as a hardener.

These products proposed by the Tombo Pencil Co., are however unsatisfactory, in that they show a tendency to shrink and deform due to evaporative loss of ethanol. The presence of an excessively high level of ethanol can also provoke a stinging sensation when the stick is applied topically to human skin, and can lead to defatting and dehydration of the skin which renders the product unsatisfactory and unacceptable for normal use. The level of perfume advocated by Tombo Pencil Co is also too high for general cosmetic use.

It has also been proposed in US patent 4 518 582 (American Cyanamid Co) to provide stable antiperspirant stick compositions containing dibenzyl monosorbitol acetal in the presence of an acidic antiperspirant active salt, such as aluminium chlorohydrex, together with 1,3-butylene glycol, anhydrous ethanol, hydroxypropyl cellulose and other ingredients. Sticks such as these, however, lose their stability if water is present, because of hydrolysis of the dibenzyl monosorbitol acetal in the presence of the acid antiperspirant salt. Sticks containing antiperspirant actives can also be cosmetically unacceptable in that they can become sticky following topical application, and they can be rejected by the consumer as being physiologically active in interfering with the body's natural function of sweating in response to excessive heat or exercise.

We have now discovered that the problems of instability shrinkage, deformity, brittleness and loss of transparency that can occur with aqueous-alcohol deodorant sticks, including those problems inherent in the products described in the American Cynamid Co and Tombi Pencil Co. references, can be avoided by careful attention to the relative amounts of water together with four key ingredients, namely a monohydric alcohol, a glycol, such as propylene glycol, a sorbitol derivative, namely 1,3,2,4-benzaldehyde sorbitol diacetal (hereinafter referred to as "dibenzyl sorbitol"), and finally a special polymer, which are combined to form the stick.

DEFINITION OF THE INVENTION

The invention accordingly provides a transparent deodorant stick for topical application to human skin which comprises:

(i) from 10 to 90% by weight of a solvent chosen from $C_2$ to $C_4$ polyhydric alkanols and mixtures thereof,

(ii) from 0 to 60% by weight of a co-solvent chosen from $C_1$ to $C_6$ monohydric alkanols, $C_2$ to $C_6$ dialkyl ketones, and mixtures thereof,

(iii) from 0 to 55% by weight of water;

(iv) from 0.5 to 5% by weight of dibenzyl sorbitol,

(v) from 0.5 to 3.5% by weight of a thickening agent chosen from chemically modified celluloses, polyacrylic acid and polyacrylic acid copolymers and mixtures thereof, and

(vi) up to 5% by weight of a perfume

provided the that the total amount of solvent, co-solvent and water together comprise at least 85% by weight of the stick, and the relative proportions by weight of the solvent, co-solvent and water in the stick are within the area designated ABCDEFGHJKL on the accompanying diagram.

DISCLOSURE OF INVENTION

2

## The Solvent

The deodorant stick according to the invention comprises a solvent chosen from $C_2$ to $C_4$ polyhydric alkanols and mixtures thereof. The polyhydric alkanol is provided primarily as a solvent for the dibenzyl sorbitol which also forms an essential ingredient of the stick, but it also functions to enhance the stability of the deodorant stick by reducing so far as is possible the problems of shrinkage, deformity and brittleness that can occur due to evaporative loss of volatile materials such as a co-solvent when present. The solvent can also function as a deodorant.

Examples of $C_2$ to $C_4$ polyhydric alkanols include glycerol and alkane diols, such as ethane-1,2-diol, propane-1,2-diol, butane-1,2-diol, butane-1,3-diol and butane-1,4-diol. The preferred diol is propane-1,2-diol.

The amount of the polyhydric alkanol present in the deodorant stick according to the invention is from 10 to 90%, preferably from 30 to 90% by weight of the stick.

## The Co-solvent

The deodorant stick according to the invention also comprises a co-solvent chosen from $C_1$ to $C_6$ monohydric alkanols, $C_2$ to $C_6$ dialkyl ketones, and mixtures thereof. The co-solvent can be present as a medium in which further to assist in dissolving dibenzyl sorbitol and also to function as a deodorant.

Examples of $C_1$ to $C_6$ monohydric alkanols are methanol, ethanol, n-propanol, isopropanol, n-butanol, isobutanol, sec-butanol, t-butanol, n-pentanol, isopentanol, t-pentanol, n-hexanol. The preferred monohydric alkanol is ethanol which is commonly available as Industrial Methylated Spirits.

Examples of dialkyl ketones include acetone, methyl ethyl ketone, methyl n-propyl ketone, diethyl ketone, hexanone-2, and hexanone-3. The preferred dialkyl ketone is methyl ethyl ketone.

The amount of the co-solvent when present in the deodorant stick according to the invention is generally from 0 to 60%, preferably from 10 to 60% by weight of the stick.

## Water

The deodorant stock according the invention also comprises water in an amount of from 0 to 55% by weight, preferably from 5 to 20% by weight of the deodorant stick.

It is also an important feature of the invention that the total amount of solvent, co-solvent and water present in the deodorant stick according to the invention together comprise at least 85% by weight of the stick.

## The Accompanying Diagram

The accompanying diagram shows the relationship in parts by weight between the solvent, co-solvent and water which is necessary to provide the best transparent deodorant sticks according to the invention.

The relative proportions by weight of solvent, co-solvent and water will together total 100 for any stick composition falling within this diagram. It is, however, understood that when formulating a deodorant stick according to the invention which will also comprise at least dibenzyl sorbitol and a thickening agent, and possibly also other ingredients, then it is necessary to take account of all the ingredients present if it is required to express the composition in terms of the percentages by weight of its ingredients.

The relative proportions by weight of solvent, co-solvent and water which comprise the transparent deodorant stick according to this preferred embodiment are accordingly within the area on the diagram which is designated ABCDEFGHJKL, the solvent forming from 9 to 85 parts by weight, the co-solvent forming from 0 to 61 parts by weight and the water forming from 0 to 56 parts by weight. The regions of the diagram outside this designated area are excluded from the scope of the invention as representing relative proportions of solvent, co-solvent and water which together would yield unsuitable or inferior deodorant sticks. They are identified on the diagram by numbers for the following reasons:

I) Excessively large amount of co-solvent can cause stinging or damage to skin when applied topically.

II) Shrinkage of stick during storage can be excessive due to evaporative loss of co-solvent.

III) Microbial spoilage can occur because of low level of co-solvent.

IV) Poor underarm feel when applied topically, due to high level of water.

V) Perfuming is difficult because of poor solubility in the presence of a very high level of water.

VI) Stick lacks rigidity and can deform easily or fracture when applied topically to the skin.

VII) Stick tends to become opaque due to evaporative losses.

The relative proportions by weight of solvent, co-solvent and water which provide better deodorant sticks according to this preferred embodiment are within the area on the diagram which is designated ABCNFGHJKL.

The relative proportions by weight of solvent, co-solvent and water which provide the best deodorant sticks according to this preferred embodiment are within the area on the diagram which is designated LMNFGK.

According to a preferred embodiment of the invention, the solvent is propane-1,2-diol and the co-solvent is ethanol, and the the relative proportions by weight of these materials together with water which are present in each of the 17 formulations of the technical Examples given later in this specification are indicated on the accompanying diagram. Also shown are the relative proportions by weight of propane-1,2-diol, ethanol and water present in the formulations taken from the four 'Prescription Examples' of the Tombo Pencil Co. reference referred to earlier. These are identified as TP1, TP2, TP3 and TP4, and it is seen that these all lie well outside the area designated as defining formulations according to the invention. It is to be also noted that Prescription Examples Nos 1, 2 and 3 describe formulations in which the total amount of solvent, co-solvent and water together comprise less than 85% by weight of the solid perfume. Furthermore, Prescription Examples No,4 describes a solid perfume whose formulation does not include a thickening agent chosen from chemically modified celluloses, polyacrylic acid and polyacrylic acid copolymers.

### Dibenzyl Sorbitol

The deodorant stick according to the invention also comprises a condensation product of benzaldehyde and sorbitol. The preferred condensation product is dibenzyl sorbitol formed by condensation of two moles of benzaldehyde and one mole of sorbitol. Dibenzyl sorbitol is included in the stick to provide structure rigidity without impairing transparency.

The amount of dibenzyl sorbitol present in the deodorant sticks according to the invention is from 0.5 to 5%, preferably from 1 to 2.5% by weight of the deodorant stick.

### Thickening Agent

The deodorant stick according to the invention also comprises a thickening agent chosen from chemically modified celluloses, polyacrylic acid and polyacrylic acid copolymers.

Examples of chemically modified celluloses include hydroxypropyl cellulose, hydroxyethyl cellulose and methyl cellulose. The preferred thickening agent is hydroxypropyl cellulose.

The amount of thickening agent present in the deodorant stick according to the invention is from 0.5 to 3.5% by weight, the amount chosen being suited to the selected thickening agent.

### OPTIONAL INGREDIENTS

The deodorant stick according to the invention can also comprise optional ingredients further to enhance its transparency, its deodorant effect, its cosmetic appeal and also to facilitate its manufacture.

The deodorant stick according to the invention can for example optionally comprise solution aids such as butyrolactone, propylene carbonate or pyrrolidone to aid the dissolution of the dibenzyl sorbitol in the solvent, particularly when a co-solvent is absent or only a very low level of co-solvent is present or when low boiling co-solvents such as methanol and acetone are employed.

The deodorant stick according to the invention can also optionally comprise emollient oils further to enhance the deodorant appeal of the stick, examples of which include isopropyl myristate, n-butyl phthalate, polyethyleneglycol 400, oleyl alcohol, as well as synthetic oils such as PPG-14 butyl ether (such as Fluid AP), Neobic A-20, PPG-5-Ceteth-20 (such as Procetyl AWS), PPG-3 Myristyl ether (such as Witconol APM) as well as lanolin, its alcohols and its esters.

The deodorant stick according to the invention can also optionally comprise a humectant such as sorbitol.

The deodorant stick according to the invention can also optionally comprise a deodorant agent in addition to the solvent and co-solvent, examples of which are 2,4,4'-trichloro-2'-hydroxydiphenyl ether, also known as Triclosan (such as Irgasan DP300), cetyltrimethylammonium bromide, cetylpyridinium chloride, benzethonium chloride, di-isobutyl phenoxy ethoxyethyldimethylbenzylammonium chloride, N-alkyl pyridinium chloride, N-cetylpyridinium bromide, benzalkonium chloride, sodium-N-lauroyl sarcosine, zinc phenol sulphonate, zinc ricinoleate, 3,7,11-trimethyl-2,6-10 deodecatriene-1-ol, also known as farnesol and a deodorant composition such as is described in US Patent 4,278,658 (Lever Brothers Company).

The deodorant stick according to the invention can also optionally comprise a cooling agent, such as

4

menthol, or derivatives thereof.

The deodorant sticks according to the invention can also comprise perfumes, usually in an amount of from 0.1 to 5% by weight of the stick, colourants, such as dyes and pigment, preservatives, such as methylparaben and propylparaben and other materials which do not detract from the transparency of the stick.

The choice of optional ingredients and the quantities employed should be such that the transparency of this deodorant stick is not impaired.

The amount of optional ingredients together with the dibenzyl sorbitol and thickening agent should together form no more than 15% by weight of the stick.

## PROCESS FOR THE MANUFACTURE OF DEODORANT STICKS

The invention also provides a process for the manufacture of the transparent deodorant sticks according to the invention, which process comprises the steps of

(i) forming a solution of dibenzyl sorbitol in the presence of a solvent chosen from $C_2$ to $C_4$ polyhydric alkanols, and mixtures thereof, with heating at a temperature of from 65° to 85°C; the solution also comprising a thickening agent chosen from chemically modified celluloses, polyacrylic acid and polyacrylic acid copolymers and mixtures thereof; and

(ii) cooling the solution and casting it into sticks.

The process of the invention can also be modified by the step of additionally incorporating into the solution a co-solvent chosen from $C_1$ to $C_6$ monohydric alkanols, $C_2$ to $C_6$ dialkyl ketones and mixtures thereof.

The process of the invention can also be modified by the further step of additionally incorporating water into the solution.

According to one embodiment of the process of the invention, a first solution is prepared by dissolving the thickening agent in water with agitation. A second solution is prepared by mixing together the solvent and the diol and then slowly adding the dibenzyl sorbitol with agitation and heating to a temperature of from 65° to 85°C, the dibenzyl sorbitol dissolving at a temperature of about 70°C.

The aqueous solution is then added to the dibenzyl sorbitol solution, and the temperature adjusted as necessary to a value of from 60°C to 70°C, followed by the addition of any remaining ingredients such as perfume as desired. Finally, the liquid mix so prepared is poured into moulds and allowed to cool and solidify to form transparent deodorant sticks.

According to a second embodiment of the process of the invention, a mixture of the solvent, diol and water is prepared and then dibenzyl sorbitol and thickening agent are added with agitation and heating to a temperature of from 65° to 85°C. The temperature is then adjusted as necessary to a value of from 60° to 70°C followed by addition of any remaining ingredients, such as perfume, as desired. Finally the liquid mix so prepared is poured into moulds and allowed to cool and solidify to form transparent deodorant sticks.

The transparent deodorant stick can be cast into any shape or size, but is preferably circular or oval in cross-section and is conveniently packed in a suitable holder with a removable cap to protect the stick and to prevent or at least reduce evaporative loss of the more volatile ingredients of the stick.

Ideally, the transparent deodorant stick according to the invention is contained in a holder adapted to enable the stick to be extended from it when required ready for use and retracted into it when not in use.

## EXAMPLES

The following examples illustrate the invention.

### Example 1

This example describes a process for the manufacture of deodorant sticks according to the invention.

Klucel M (4.9g) was dissolved at room temperature in water (390g) by dispersion of the solid in the vortex of the rapidly stirred liquid. Propane-1,2-diol (97.6g) and Industrial Methylated Spirits (488g) were charged into a separate vessel equipped with a paddle stirrer and water condenser. The liquid was stirred sufficiently rapidly to guarantee the immediate even dispersion of the dibenzyl sorbitol (9.8g) as it was added. The vessel was then heated to a gentle reflux (78°C± 5°C). It was observed that most dissolution occurred at approximately 70°C but that any lumps or dibenzyl sorbitol caused by imperfect dispersion took rather longer to dissolve.

Once both solutions were perfectly uniform the aqueous solution was added to the stirred alcoholic solution. After 1 minute the rate of stirring was reduced. When a uniform solution was again achieved the solution temperature was reduced to 65°C ± 5°C (being 5 to 10°C above the gellation temperature) and the perfume (9.7g) was added and mixed in.

The mixture was finally poured into moulds which were capped, inverted and allowed to cool under ambient conditions.

The transparent deodorant sticks so prepared had the following composition:

|  | % w/w |
|---|---|
| Water | 39 |
| Industrial Methylated Spirits | 48.8 |
| Propane-1,2-diol | 9.76 |
| Dibenzyl sorbitol | 0.98 |
| Hydroxypropyl cellulose | 0.49 |
| Perfume | 0.97 |

Example 2

The procedure described in Example 1 was repeated using different quantities of the major ingredients to form transparent deodorant sticks according to the invention having the following composition:

|  | % w/w |
|---|---|
| Water | 29 |
| Propane-1,2-diol | 68 |
| Dibenzyl sorbitol | 1.5 |
| Hydroxypropyl cellulose | 0.5 |
| Perfume | 1 |

Example 3

This example describes an alternative process for the manufacture of deodorant sticks according to the invention.

A suitable vessel was equipped with a paddle stirrer and water condenser. Water (48g), industrial methylated spirits (288g) and propane-1,2-diol (624g) were charged into the vessel. The stirrer was set fast enough to cause a large vortex and the dibenzyl sorbitol (20g) and Klucel (10g) added at such a rate as to allow immediate and complete dispersion to occur.

The temperature was raised to 78°C ± 5°C whilst maintaining the stirring. Most of the solid was observed to dissolve at 70°C ± 5°C. Heating and stirring was continued until dissolution was complete. Once a homogeneous solution was achieved the stirring rate was reduced and the temperature was then reduced to 65°C ± 5°C (being 5-10°C above the gellation temperature) and the perfume (10g) was then added and mixed in.

The mixture was finally cast into sticks which were then capped inverted and allowed to cool under ambient conditions.

The transparent deodorant sticks so prepared had the following composition:

|  | % w/w |
|---|---|
| Water | 4.8 |
| Industrial Methylated Spirits | 28.8 |
| Propane-1,2-diol | 62.4 |
| Dibenzyl sorbitol | 2 |
| Hydroxypropyl cellulose | 1 |
| Perfume | 1 |

Example 4

The procedure described in Example 3 was repeated using different quantities of the major ingredients to form transparent deodorant sticks according to the invention having the following composition:

|  | % w/w |
|---|---|
| Water | 19.3 |
| Industrial Methylated Spirits | 47.4 |
| Propane-1,2-diol | 29.6 |
| Dibenzyl sorbitol | 1.5 |
| Hydroxypropyl cellulose | 1.2 |
| Perfume | 1 |

Examples 5 & 6

The procedure described in Example 3 was repeated using different quantities of the major ingredients to form transparent deodorant sticks according to the invention having the following composition:

|  | % w/w | |
|---|---|---|
|  | Example 5 | 6 |
| Water | 10.6 | 4.8 |
| Industrial Methylated Spirits | 10.6 | 57.8 |
| Propane-1,2-diol | 75.1 | 33.7 |
| Dibenzyl sorbitol | 1.5 | 1.5 |
| Hydroxypropyl cellulose | 1.2 | 1.2 |
| Perfume | 1 | 1 |

## Examples 7 to 14

The following examples illustrate alternative transparent deodorant sticks according to the invention:

| | EXAMPLE | | | | | | | | | | |
|---|---|---|---|---|---|---|---|---|---|---|---|
| | 7 | 8 | 9 | 10 | 11 | 12 | 13 | 14 | 15 | 16 | 17 |
| | (PARTS BY WEIGHT) | | | | | | | | | | |
| Water | 30 | 30 | 20 | 10 | 47 | 9 | 5 | 45 | 40 | - | - |
| Industrial Methylated Spirits | 30 | 50 | 20 | 40 | 33 | 36 | 17 | 20 | 30 | 30 | 60 |
| Propane-1,2-diol | 40 | 20 | 60 | 50 | 20 | 46 | 78 | 35 | 30 | 70 | 40 |
| Dibenzyl sorbitol | 1.5 | 1.5 | 1.5 | 1.5 | 1 | 1.5 | 1 | 1.5 | 1 | 2 | 2 |
| Hydroxypropyl cellulose | 0.5 | 0.5 | 0.5 | 0.5 | 1 | 1.5 | 1 | 1.5 | 0.5 | 1 | 1 |
| Perfume | 1 | 1 | 1 | 1 | 1 | 1 | 1 | 1 | 1 | 1 | 1 |
| Zinc phenol sulphonate | - | - | - | - | - | 5 | - | - | - | - | - |
| Irgasan DP 300 | 0.1 | - | - | - | - | - | - | 0.1 | - | - | - |
| Farnesol | - | - | - | - | - | - | 0.3 | - | - | - | - |
| Fluid AP | - | - | - | - | 5 | - | - | 5 | - | - | - |
| PEG 400 | - | 5 | - | - | - | - | - | - | - | - | - |

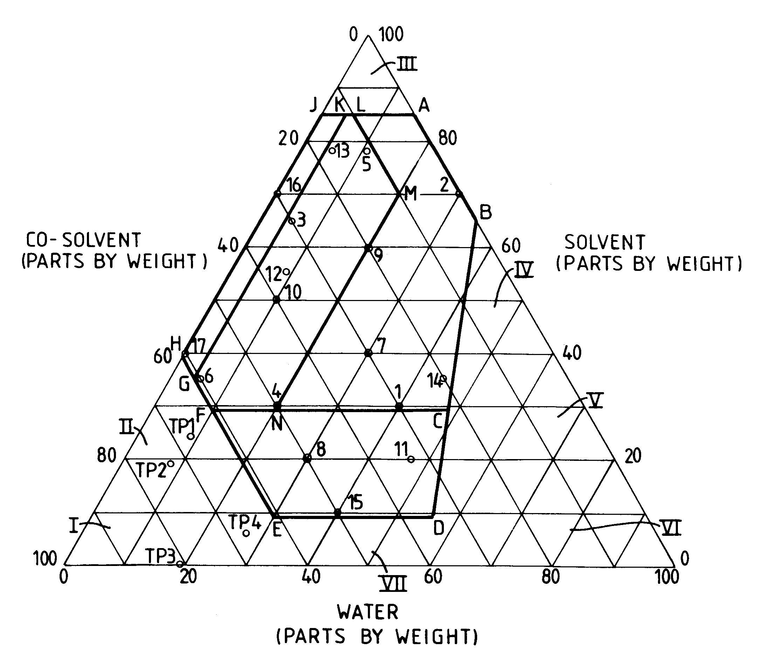

## Claims

1. A transparent deodorant stick for topical application to human skin which comprises:
   (i) from 10 to 90% by weight of a solvent chosen from $C_2$ to $C_4$ polyhydric alkanols and mixtures thereof,

(ii) from 0 to 60% by weight of a co-solvent chosen from $C_1$ to $C_6$ monohydric alkanols, $C_2$ to $C_6$ dialkyl ketones, and mixtures thereof,

(iii) from 0 to 55% by weight of water;

(iv) from 0.5 to 5% by weight of dibenzyl sorbitol,

(v) from 0.5 to 3.5% by weight of a thickening agent chosen from chemically modified celluloses, polyacrylic acid and polyacrylic acid copolymers and mixtures thereof, and

vi) up to 5% by weight of a perfume

provided that the total amount of solvent, co-solvent and water together comprise at least 85% by weight of the stick, and the relative proportions by weight of the solvent, co-solvent and water in the stick are within the area designated ABCDEFGHJKL on the accompanying diagram.

2. A deodorant stick according to claim 1, in which the solvent is propane-1,2-diol.

3. A deodorant stick according to claim 1 or 2, in which the solvent forms from 30 to 90% by weight of the stick.

4. A deodorant stick according to claim 1, 2 or 3, in which the co-solvent is ethanol.

5. A deodorant stick according to claim 1, 2 or 3, in which the co-solvent is isopropanol.

6. A deodorant stick according to claim 1, 2 or 3, in which the co-solvent is methyl ethyl ketone.

7. A deodorant stick according to any preceding claim, in which the co-solvent forms from 10 to 60% by weight of the stick.

8. A deodorant stick according to any preceding claim, in which the water forms from 5 to 20% by weight of the stick.

9. A deodorant stick according to any preceding claim, in which dibenzyl sorbitol forms from 1 to 2.5% by weight of the stick.

10. A deodorant stick according to any preceding claim, in which the thickening agent is hydroxypropylcellulose.

11. A deodorant stick according to any preceding claim, in which the relative proportions by weight of the solvent, co-solvent and water in the stick are within the area designated ABCNFGHJKL on the accompanying diagram.

12. A deodorant stick according to any of claims 1 to 10, in which the relative proportions by weight of the solvent, co-solvent and water in the stick are within the area designated LMNFGK on the accompanying diagram.

13. A deodorant stick according to any preceding claim, which comprises:

(i) from 10 to 90% by weight of propane-1,2-diol,

(ii) from 0 to 60% by weight of ethanol, and

(iii) from 0 to 55% by weight of water,

provided that the relative proportions by weight of propane-1,2-diol, ethanol and water in the stick are within the area designated ABCDEFGHJKL on the accompanying diagram.

14. A process for the manufacture of the transparent deodorant sticks according to any of claims 1 to 13, which process comprises the steps of:

(i) forming a solution of dibenzyl sorbitol in the presence of a solvent chosen from $C_2$ to $C_4$ polyhydric alkanols and mixtures thereof, with heating at a temperature of from 65° to 85°C; the solution also comprising a thickening agent chosen from chemically modified celluloses, polyacrylic acid and polyacrylic acid copolymers and mixtures thereof; and

(ii) cooling the solution and casting it into sticks.

**15.** A process according to claims 14, which comprises the step of additionally incorporating into the solution a co-solvent chosen from $C_1$ to $C_6$ monohydric alkanols, $C_2$ to $C_6$ dialkyl ketones and mixtures thereof.

**16.** A process according to claim for 14 or 15, which comprises the step of additionally incorporating water into the solution.

**Patentansprüche**

**1.** Durchsichtiger Deodorantstift zur lokalen Anwendung auf der menschlichen Haut, welcher umfaßt:

(i) 10 bis 90 Gew.-% eines Lösungsmittels, ausgewählt aus $C_2$-bis $C_4$-, mehrere Hydroxylgruppen enthaltende Alkanole und Gemische davon,

(ii) 0 bis 60 Gew.-% eines Hilfslösungsmittels, ausgewählt aus $C_1$- bis $C_6$-, einwertige Alkanole, $C_2$-bis $C_6$-Dialkylketone, und Gemische davon,

(iii) 0 bis 55 Gew.-% Wasser,

(iv) 0,5 bis 5 Gew.-% Dibenzylsorbit,

(v) 0,5 bis 3.5 Gew.-% eines Verdickungsmittels, ausgewählt aus chemisch modifizierten Cellulosen, Polyacrylsäure und Polyacrylsäure-Copolymeren und Gemische davon, und

(vi) bis zu 5 Gew.-% eines Parfüms

vorausgesetzt, daß die Gesamtmenge an Lösungsmittel, Hilfslösungsmittel und Wasser zusammen mindestens 85 Gew.-% des Stifts umfassen und, daß die relativen Gewichtsverhältnisse von Lösungsmittel, Hilfslösungsmittel und Wasser im Stift, innerhalb der mit ABCDEFGHJKL gekennzeichneten Fläche in dem beigefügten Diagramm liegen.

**2.** Deodorantstift nach Anspruch 1, worin das Lösungsmittel Propan-1,2-diol ist.

**3.** Deodorantstift nach Anspruch 1 oder 2, worin das Lösungsmittel 30 bis 90 Gew.-% des Stifts beträgt.

**4.** Deodorantstift nach Anspruch 1, 2 oder 3, worin das Hilfslösungsmittel Ethanol ist.

**5.** Deodorantstift nach Anspruch 1, 2 oder 3, worin das Hilfslösungsmittel Isopropanol ist.

**6.** Deodorantstift nach Anspruch 1, 2 oder 3, worin das Hilfslösungsmittel Methylethylketon ist.

**7.** Deodorantstift nach einem der vorhergehenden Ansprüche, worin das Hilfslösungsmittel 10 bis 60 Gew.-% des Stifts beträgt.

**8.** Deodorantstift nach einem der vorhergehenden Ansprüche, worin das Wasser 5 bis 20 Gew.-% des Stifts beträgt.

**9.** Deodorantstift nach einem der vorhergehenden Ansprüche, worin Dibenzylsorbit 1 bis 2,5 Gew.-% des Stifts beträgt.

**10.** Deodorantstift nach einem der vorhergehenden Ansprüche, worin das Verdickungsmittel Hydroxypropylcellulose ist.

**11.** Deodorantstift nach einem der vorhergehenden Ansprüche, worin die relativen Gewichtsverhältnisse von Lösungsmittel, Hilfslösungsmittel und Wasser im Stift innerhalb der mit ABCNFGHJKL gekennzeicheneten Fläche auf dem beiliegenden Diagramm liegen.

**12.** Deodorantstift nach einem der Ansprüche 1 bis 10, worin die relativen Gewichtsverhältnisse von Lösungsmittel, Hilfslösungsmittel und Wasser im Stift innerhalb der mit LMNFGK gekennzeichneten Fläche auf dem beiliegenden Diagramm liegen.

**13.** Deodorantstift nach einem der vorhergehenden Ansprüche, welcher umfaßt:

(i) 10 bis 90 Gew.-% Propan-1,2-diol,

(ii) 0 bis 60 Gew.-% Ethanol, und

(iii) 0 bis 55 Gew.-% Wasser,

vorausgesetzt, daß die relativen Gewichtsverhältnisse von Propan-1,2-diol, Ethanol und Wasser im Stift innerhalb der mit ABCDEFGHJKL gekennzeichneten Fläche auf dem beiliegenden Diagramm liegen.

14. Verfahren zur Herstellung eines durchsichtigen Deodorantstifts nach einem der Ansprüche 1 bis 13, wobei das Verfahren folgende Schritte umfaßt:

(i) Bildung einer Lösung von Dibenzyl-Sorbit in Anwesenheit eines Lösungsmittels ausgewählt aus $C_2$- bis $C_4$-, mehrere Hydroxylgruppen enthaltenden Alkanolen und Gemischen davon, unter Erwärmen auf eine Temperatur zwischen 65°C und 85°C; wobei die Lösung auch ein Verdickungsmittel enthält, ausgewählt aus chemisch modifizierten Cellulosen, Polyacrylsäure und Polyacrylsäure-Copolymeren und Gemischen davon; und

(ii) Abkühlen der Lösung und Gießen derselben in Stifte.

15. Verfahren nach Anspruch 14, welches den Schritt einer zusätzlichen Einarbeitung in die Lösung eines Hilfslösungsmittels, das aus $C_1$- bis $C_6$-, einwertigen Alkanolen, $C_2$- bis $C_6$- Dialkyl-Ketonen und Gemischen davon ausgewählt ist, umfaßt.

16. Verfahren nach Anspruch 14 oder 15, welches den Schritt der zusätzlichen Einarbeitung in die Lösung von Wasser, umfaßt.

## Revendications

1. Un bâton désodorisant transparent pour une application topique sur la peau de l'être humain comprenant :

(i) de 10 à 90% en masse d'un solvant choisi à partir l'alkanols polyhydriques en $C_2$-$C_4$ et de mélanges de ceux-ci,

(ii) de 0 à 60% en masse d'un co-solvant choisi à partir d'alkanols monohydriques en $C_1$ -$C_6$, de dialkyl cétones en $C_2$-$C_6$, et de mélanges de ceux-ci,

(iii) de 0 à 55% en masse d'eau,

(iv) de 0,5 à 5% en masse de sorbitol dibenzylique,

(v) de 0,5 à 3,5% en masse d'un agent épaississant choisi à partir de celluloses modifiées chimiquement, d'acide polyacrylique et de copolymères d'acide polyacrylique et de mélanges de ceux-ci, et

(vi) jusqu'à 5% en masse d'un parfum

à condition que la quantité totale de solvant, de co-solvant et d'eau pris ensemble compose au moins 85% en masse du bâton, et que les proportions relatives en masse du solvant, du co-solvant et de l'eau dans le bâton soient comprises à l'intérieur de la zone désignée ABCDEFGHIJKL du dessin d'accompagnement.

2. Un bâton désodorisant selon la revendication 1, dans lequel le solvant est du propane-1,2-diol.

3. Un bâton désodorisant selon la revendication 1 ou 2, dans lequel le solvant forme de 30 à 90% en masse du bâton.

4. Un bâton désodorisant selon la revendication 1, 2 ou 3, dans lequel le co-solvant est de l'éthanol.

5. Un bâton désodorisant selon la revendication 1, 2 ou 3, dans lequel le co-solvant est de l'isopropanol.

6. Un bâton désodorisant selon la revendication 1, 2 ou 3, dans lequel le co-solvant est de la méthyl éthyl cétone.

7. Un bâton désodorisant selon l'une des revendications précédentes, dans lequel le co-solvant forme de 10 à 60% en masse du bâton.

8. Un bâton désodorisant selon l'une des revendications précédentes, dans lequel l'eau forme de 5 à 20% en masse du bâton.

9. Un bâton désodorisant selon l'une des revendications précédentes, dans lequel le sorbitol dibenzylique forme de 1 à 2,5% en masse du bâton.

**10.** Un bâton désodorisant selon l'une des revendications précédentes, dans lequel l'agent épaississant est de l'hydroxypropylcellulose.

**11.** Un bâton désodorisant selon l'une des revendications précédentes, dans lequel les proportions relatives en masse du solvant, du co-solvant et de l'eau dans le bâton sont comprises à l'intérieur de la zone désignée ABCDEFGHIJKL sur le dessin d'accompagnement.

**12.** Un bâton désodorisant selon l'une des revendications 1 à 10, dans lequel les proportions relatives en masse du solvant, du co-solvant et de l'eau dans le bâton sont comprises à l'intérieur de la zone désignée LMNFGK sur le dessin d'accompagnement.

**13.** Un bâton désodorisant selon l'une des revendications précédentes, comprenant:
(i) de 10 à 90% en masse de propane-1,2-diol,
(ii) de 0 à 60% en masse d'éthanol, et
(iii) de 0 à 55% en masse d'eau,
à condition que les proportions relatives en masse du propane-1,2-diol, de l'éthanol et de l'eau dans le bâton soient comprises à l'intérieur de la zone désignée ABCDEFGHIJKL sur le dessin d'accompagnement.

**14.** Un procédé pour la fabrication des bâtons désodorisants transparents selon l'une des revendications 1 à 13, ce procédé comprenant les étapes consistant à :
(i) former une solution de sorbitol dibenzylique en présence d'un solvant choisi à partir d'alkanols polyhydriques en C2-C4 et de mélanges de ceux-ci, avec un chauffage à une température allant de 65° à 85°C ; la solution comprenant également un agent épaississant choisi à partir de celluloses modifiées chimiquement, d'acide polyacrylique et de copolymères d'acide polyacrylique et de mélanges de ceux-ci ; et
(ii) refroidir la solution et à la mouler en bâtons.

**15.** Un procédé selon la revendication 14, comprenant l'étape consistant à ajouter en outre à la solution un co-solvant choisi à partir d'alkanols monohydriques en $C_1$-$C_6$, de dialkyl cétones en $C_2$-$C_6$ et de mélanges de ceux-ci.

**16.** Un procédé selon la revendication 14 ou 15, comprenant l'étape consistant à incorporeren outre de l'eau à la solution.

12